# EUROPEAN PATENT APPLICATION

(11) **EP 4 458 972 A1**
(43) Date of publication of application: **06.11.2024**
(21) Application number: 23765746.5
(22) Date of filing: 16.02.2023
(51) Int. Cl.: C12N 15/60, C12N 9/88, C12N 15/70, C12N 1/21, C12P 13/02

(54) **USE OF ASPARTATE DECARBOXYLASE IN FERMENTATION PRODUCTION OF VITAMIN B5**

(30) Priority: 07.03.2022 CN 202210214711
(71) Applicant: Institute Of Microbiology, Chinese Academy Of Sciences, Beijing 100101 (CN)
(72) Inventor: WEN, Tingyi, Beijing 100101 (CN); LIU, Shuwen, Beijing 100101 (CN); LI, Zhongcai, Beijing 100101 (CN); SUN, Jiahui, Beijing 100101 (CN); DENG, Aihua, Beijing 100101 (CN); ZHANG, Yun, Beijing 100101 (CN)
(74) Representative: Strych, Sebastian
(86) International application number: PCT/CN2023/076309
(87) International publication number: WO 2023/169168

(57) **Abstract**

The present application relates to the field of microorganisms and specifically relates to highly active aspartate decarboxylases for the production of vitamin B5. In the present application, the L-aspartate α-decarboxylase derived from *Bacillus licheniformis* was screened, the PanD with significantly higher activity which catalyze the production of β-alanine than other orgins of PanD. The engineering bacterium for the fermentative production of vitamin B5 was constructed by applying PanD derived from *B. licheniformis.* The bottleneck of β-alanine metabolism in the biosynthesis of vitamin B5 was lifted. Compared with the highly polluting chemical method for the production of vitamin B5, the biological method for the production of vitamin B5 of the present application has the advantages of renewable raw materials, easy treatment and resource utilization of waste residue, waste water and waste gas, and thus can be used in practice for the industrial production of vitamin B5, which is of significant application value.

## Description

### Technical Field

The present application relates to the field of microorganisms and specifically relates to the application of aspartate decarboxylase in the fermentation production of vitamin B5.

### Background

Vitamin B5 (VB5), also known as D-pantothenic acid, is a water-soluble vitamin, is a component of coenzyme A and acyl carrier protein, as a cofactor of more than 70 kinds of enzymes, the vitamin B5 participates in the metabolism of sugar, fat, protein and energy, and has an important physiological metabolism regulation. VB5 is mainly used in animal feed additives, food additives and pharmaceutical raw materials, with the discovery of new functions of VB5 and the expansion of application field, its market demand still tend to increase steadily.

China is the largest country in the production and export of VB5, the industrial production method of VB5 is a chemical synthesis. Enterprises basically use the Isobutyraldehyde-formaldehyde-hydrocyanic acid method to synthesize DL-pantolactone, the DL-pantolactone is further split by a chemical or enzymatic method to obtain L-pantolactone. Finally, the β-alanine produced by using acrylonitrile as a raw material and the L-pantolactone synthesizes VB5. The main raw materials for chemical synthesis of VB5 are flammable, explosive, and highly toxic. Cyanic wastewater will be produced in the production process, and the wastewater is difficult to treat, leading to VB5 becoming a heavy pollution industry.

In recent years, China's economic development has entered into a new state of green environmental protection, and the impact of environmental protection on the VB5 industry has gradually emerged. Under large-scale and high-intensity environmental protection and management, the production of high-pollution VB5 enterprises have been limited and even ceased, and the market supply is short and the price is skyrocketed, which has restricted the health development of the downstream feed, food and pharmaceutical industries. Such a supply and demand situation will continue for a long time until there is a significant improvement in the highly polluting VB5 production technology, therefore, the innovation of VB5 green manufacturing technology for VB5 is urgent.

The microbial fermentation method for VB5 production not only use renewable glucose as raw material, but also waste residues, waste water and waste gas formed in the production process are easy to be treated and utilized in a resourceful way, thereby effectively solving the problem of high pollution in the VB5 industry. The metabolic pathway of VB5 synthesis by microorganisms using glucose has a complex regulatory mechanism and the fermentation yield is extremely low. β-alanine is used as a C3 substrate to synthesize VB5 with D-pantoic acid. β-alanine is produced from aspartic acid catalyzed by L-aspartate α-decarboxylase encoded by the *panD* gene. The initially translated and synthesized *panD* is a zymogen having no catalytic activity, and the zymogen spontaneously shears at the Gly-Ser bond to produce two sub-units, of which the N-terminal subunit containing the pyruvoyl group has a catalytic effect. The mature *panD* pyruvyl group forms a transition intermediate state with the substrate, which is susceptible to aminotransfers that result in irreversible loss of enzyme activity. In addition, the accumulation of β-alanine is also regulated by the concentration of the downstream metabolite of VB5, coenzyme A. The protein complex formed by coenzyme A with PanD/PanZ negatively regulates the expression of PanD by feedback. PanD not only has a slow maturation process of post-translational modification, but also has the problems of catalytic inactivation and feedback inhibition, resulting in a very low synthetic efficiency of β-alanine, the C3 precursor of VB5, limiting the efficient synthesis of VB5. In order to improve the fermentation yield of VB5, a large amount of beta-alanine needs to be supplemented exogenously in the fermentation medium (Sahm, H., et al., (1999) Appl Environ Microb, 65, 1973-1979; Dusch, N., et al. 1530-1539; Zhang, B., et al., (2019) Food Chemistry, 294, 267-275.). Thus, the biosynthesis of β-alanine is a metabolic bottleneck in the production of VB5 by fermentation.

### Summary

In view of the above, in order to break through the metabolic bottleneck for highly efficient β-alanine synthesis, the inventors screened 16 key enzymes L-aspartate α-decarboxylase of different genera origins and with large evolutionary differences. L-Aspartate α-decarboxylase is encoded by the *panD* gene, which catalyzes the decarboxylation of L-aspartate to produce β-alanine.

In order to realize the purpose of the invention, the present application provides the following technical solutions:
In a first aspect, the present application provides an application of enhanced expression of a L-aspartate α-decarboxylase gene *panD* in the production of vitamin B5;
the L-aspartate α-decarboxylase is derived from *Bacillus licheniformis.*
In some embodiments of the present application, the L-aspartate α-decarboxylase gene *panD* has:
   (I) a nucleotide sequence shown as SEQ ID No. 3; or
   (II) a nucleotide sequence obtained by substituting, deleting or adding one or more bases to the nucleotide sequence shown as (I) and having the same or similar functions as the nucleotide sequence shown as (I); or
   (III) a nucleotide sequence having at least 80% homology to the nucleotide sequence shown as (I) or (II).

In some embodiments of the present application, further comprising:
(1) inserting a strong promoter and/or a strong RBS into the *avtA* gene, wherein the strong promoter is PPL and the strong RBS is BCD2; and/or
(2) expressing an *ilvGM* gene derived from E. coli BL21; and/or
(3) a *panB* gene, a *panC* gene and/or a *panE* gene with increased copy number.

In a second aspect, the present application also provides expression vectors comprising the L-aspartate α-decarboxylase gene *panD*;
the L-aspartate α-decarboxylase is derived from *Bacillus licheniformis.*

In some embodiments of the present application, the expression vector further comprises:
(i) a strong promoter and/or a strong RBS; and/or
(ii) an *ilvGM* gene derived from E. coli BL21; and/or
(iii) a *panB* gene, a *panC* gene and/or a *panE* gene with increased copy number.

In a third aspect, the present application also provides host, expressing the L-aspartate α-decarboxylase *gene panD* derived from *Bacillus licheniformis.*

In some embodiments of the present application, the host further comprises:
(i) a strong promoter and/or a strong RBS; and/or
(ii) an *ilvGM* gene derived from E. coli BL21; and/or
(iii) a *panB* gene, a *panC* gene and/or a *panE* gene with increased copy number.

In some embodiments of the present application, the host is transfected or transformed with the expression vector according to claim 4 or 5;
preferably, the host is derived from E. coli,
preferably, the host is derived from E. coli K12, and
more preferably, the host is derived from E. coli K12 MG1655 strain.

In a fourth aspect, the present application also provides the application of the expression vector, the host in the production of vitamin B5.

In a fifth aspect, the present application also provides a method for the production of vitamin B5. The host is used as a fermentation strain without the addition of β-alanine, fermented, the fermentation broth is collected, and supernatant is centrifuged to obtain vitamin B5.

The present application discloses an aspartic acid decarboxylase with high activity and a method for its application in the production of vitamin B5. The present application screened a L-aspartate α-decarboxylase derived from *Bacillus licheniformis,* which has the activity of catalyzing and producing β-alanine is obviously higher than that of *panD* from other sources. The *panD* derived from B. licheniformis was applied to construct an engineering bacterium for the fermentation production of vitamin B5, which released the metabolic bottleneck of β-alanine for biosynthesis of vitamin B5. Compared with the highly polluting chemical method of vitamin B5 production, the biological method of vitamin B5 production of the present application has the advantages of renewable raw materials, easy treatment and resource utilization of waste residue, waste water and waste gas etc. Therefore, it can be used for the industrial production of vitamin B5 in practice, and has an important application value.

### Detailed Description of Embodiments

The present application discloses the application of aspartic acid decarboxylase in the fermentation production of vitamin B5. The skilled in the art can refer to the contents of this paper and improve the process parameters appropriately. It should be noted that all similar substitutions and modifications are obvious to the skilled in the art, and they are all considered to be included in the present application. The method and application of the present application have been described through preferred embodiments, and relevant personnel can obviously modify or appropriately change and combine the method and application described herein without deviating from the content, spirit and scope of the present application to achieve and apply the technology of the present application.

The *panD* genes of the present application are derived from *Bacillus amyloliquefaciens, Bacillus subtilis, Bacillus licheniformis, Chlorobium phaeobacteroides, Corynebacterium efficiens, Corynebacterium glutamicum, Corynebacterium marinum, Escherichia coli, Haloquadratum walsbyi, Hydrothermal vent metagenome, Methanocaldococcus jannaschii, Magnetospirillum magneticum , Metarhizium robertsii, Mine drainage metagenome, Rhodopirellula baltica and Thermotoga maritima.*

In terms of screening for highly efficient L-aspartate α-decarboxylases, the present invention uses the same bicistronic design element BCD2 (Nature Methods, 2013, 10(4):354-360) to regulate the translational initiation levels of the 16 different orgins of L-aspartate α-decarboxylases mentioned above. The BCD element introduces a leading cis-trans sequence in front of the exogenous structural gene, and when ribosome passes through the cis-trans and occupies the next cis-trans RBS sequence, it can avoid forming a stem-loop structure with the coding sequence of the front end of the gene, thus avoiding the translation blockage of part of the *panD* genes. The translation intensity of BCD is highly correlated with the affinity between RBS core sequence and ribosome subunit, and has little correlation with gene coding sequence, thus avoiding the interference of 16 different *panD* gene sequences on the same translation initiation element.

In terms of screening for highly efficient L-aspartate α-decarboxylase, the present application ligates the above 16 BCD2-*panD* sequences to plasmids and constructs 16 recombinant plasmids, pET28a-BCD2-*panD*, which use the same promoter to regulate transcription. The plasmid vector used in the present application can be a pET series vector, such as pET28a, pET32a, pET3, etc; it can also be a pQE series vector or other E. coli expression vector. The promoter of the present application can be T7 promoter, etc.

In the present invention, the above recombinant plasmids were transformed into a derivative strain of Escherichia coli B, including BL21, BL21-Codonplus (RIL) , BL21(DE3), BL21 Star , C41(DE3), BL21(DE3)pLys S/E, BL21-CodonPlus (DE3) strains, Origami(DE3) strains, Rosetta-gammi (DE3), etc, to obtain whole-cell catalytic engineering bacteria. Recombinant vectors are usually constructed by restriction endonuclease digestion and T4 ligase ligation of the obtained target gene and vector. The recombinant vector can be transformed into the host cells by chemical transformation with conventional calcium chloride or electroporation transformation in molecular biology experiments, to obtain the engineering bacterium that can be used for whole-cell catalysis.

The present application uses a whole cell catalysis method to screen efficient L-aspartate α-decarboxylase. In the whole-cell catalytic process of the present application, the bacterial cells are first cultured in liquid medium and L-aspartate α-decarboxylase expression is induced at the appropriate time. The medium used for the growth of the engineering bacteria can be a rich medium or can be an inorganic salt medium. The medium contains a carbon source, a nitrogen source, inorganic ions, antibiotics and other nutritional factors. As the carbon source, sugars such as glucose, lactose, galactose, etc can be used; alcohols such as glycerol, mannitol, etc; and organic acids such as gluconic acid, citric acid, succinic acid, etc can be used. As carbon source, inorganic nitrogen sources such as ammonia, ammonium sulfate, ammonium phosphate, and ammonium chloride etc can be used; organic nitrogen sources such as corn syrup, soybean meal hydrolysate, hair powder, yeast extract, peptone and other organic nitrogen sources can also be used. Inorganic ions include one or more of ions such as iron, calcium, magnesium, manganese, molybdenum, cobalt, copper, and potassium. Other nutritional factors include vitamins such as vitamin B1, pyridoxal, biotin and other vitamins.

The culture process is suitable to implement the culture under aerobic conditions for about 5-48 hours, the culture temperature is usually controlled at 25-45°C, and the pH is usually controlled at 5-8. The at least one inducer selected from IPTG, lactose, and allo-lactose is started to be added from the culture for 3-40 hours, and the addition of the inducer can be a one-time, intermittent, or continuous addition, and the addition amount of the inducer is 0.01-1 mmol.

The substrate L-aspartic acid, typically preferably L-aspartic acid, L-aspartate sodium salt, L-aspartate potassium salt, L-aspartate ammonium salt etc, is added in a one-time manner, after the inducer is added for 0.5 to 30 hours.

During the L-aspartic acid decarboxylation reaction for the production of β-alanine, the pH of the catalytic solution continues to rise, and acid needs to be added to maintain the pH in a range that is favorable for whole-cell catalysis, usually pH is more than 4.0, preferably more than 5.0, more preferably more than 5.5, and usually pH is less than 8.0, preferably less than 7.5, more preferably less than 6.8. The acid used herein is L-aspartic acid. It may be supplemented in the form of a solid powder, suspension or solution; it may be supplemented intermittently or continuously to maintain the pH in the above range, or it may be supplemented to maintain the pH at a constant value by feedback from the pH electrode signal of the bioreactor.

The temperature of the catalytic reaction is typically between 25°C and 60°C, preferably between 30°C and 45°C. The temperature during the catalytic process may be set at a fixed value within the above range or may vary from low to high.

The present application further applies the screened efficient L-aspartate α-decarboxylase to construct an engineering bacterium for the production of vitamin B5 by the fermentation method, which removes the β-alanine metabolic bottleneck for the biosynthesis of vitamin B5.

The E. coli mentioned in the present application for VB5 production by fermentation expresses *panB, panC* and *panE* genes on the terminal synthesis pathway of VB5. The *panB* gene of E. coli encodes a ketopantothenate hydroxymethyltransferase which catalyzes the addition of a methyl group to the substrate α-ketoisovaleric acid to form ketopantoate. Ketopantoate is reduced to pantoic acid by ketopantothenate reductase encoded by the *panE* gene. The pantothenate synthase encoded by the *panC* gene further catalyzes the condensation of pantothenate and β-alanine to form VB5.

The genome of E. coli K12 MG1655 was used as a template for PCR amplification of the *panB*C gene. The strong promoter Ptrc was designed to be introduced on the amplification primer, and BamHI and SphI restriction endonuclease sites were designed on both ends of the primer. The Ptrc-panBC product amplified by PCR was identified and recovered by gel electrophoresis, and then was double digested using BamHI and SphI. The pACYC184 plasmid were simultaneously double digested with restriction endonucleases BamHI and SphI. The double-cleaved Ptrc-panBC and pACYC184 plasmids were recovered by gel electrophoresis, ligated with T4 ligase, and the ligation products were chemically transformed into E. coli DH5α competent cells, which were resuscitated for 1 hour and coated on the chloramphenicol plates. The coated plates were placed in a 37°C incubator for 12 hours, single colonies were picked for passaging, and the recombinant plasmids were extracted and sequenced to obtain the correct recombinant plasmid pACYC184-Ptrc-panBC.

The genome of E. coli K12 MG1655 was used as a template for PCR amplification of the *panE* gene. The strong promoter PJ23119 was designed to be introduced on the amplification primer, and SphI and BsaBI restriction endonuclease sites were designed on both ends of the primer. The PJ23119-panE product obtained from PCR amplification was identified and recovered by gel electrophoresis, and then was double digested with BamHI and SphI. The pACYC184-Ptrc-panBC plasmid were simultaneously double digested with restriction endonucleases BamHI and SphI. The double digested PJ23119-panE and pACYC184-Ptrc-panBC plasmids were recovered by gel electrophoresis, ligated with T4 ligase, and the ligation products were chemically transformed into E. coli DH5α competent cells, which were resuscitated for 1 hour and coated on the chloramphenicol plates. The coated plates were placed in a 37°C incubator for 12 hours, single colonies were picked for passaging, and the recombinant plasmids were extracted and sequenced to obtain the correct recombinant plasmid pACYC184-Ptrc-panBC-PJ23119-panE that overexpresses the gene for the vitamin B5 terminal synthesis pathway.

The *ilvG* gene of E. coli K12 MG1655 was inactivated by mutation, and the present application introduced the active *ilvG* gene of E. coli BL21 to improve the synthesis and supply of the precursor of VB5. The present application inserted the *ilvG⁺M gene* derived from E. coli BL21 on the chromosome of E. coli K12 MG1655, and used the trc strong promoter to regulate the transcription initiation of *ilvG*⁺*M,* and used the terminator Ter to regulate the transcription termination of *ilvG⁺M.* The insertion site of the *ilvG⁺M* gene in the chromosome was the coding sequence of the *avtA* gene, which leads to inactivation of *AvtA* and weakens the synthesis of valine, thereby weakening the competitive pathway for VB5 and favoring VB5 biosynthesis. The engineering bacterium E. coli MG1655 *avtA:ilvG*⁺*M* was constructed to enhance the synthesis pathway of the VB5 precursor acetolactate and weaken the valine competition pathway.

Three more active *panD* genes screened above were integrated on the *avtA* gene of E. coli K12 MG1655, which derived from *Bacillus subtilis, Bacillus licheniformis* and *Corynebacterium glutamicum,* respectively. These three genes use the same strong promoter PPL and the same BCD2 to regulate transcription and translation initiation, respectively.

The above constructed recombinant plasmid pACYC184-Ptrc-*panB*C-PJ23119-*panE* was transformed into Engineering Bacteria E. coli MG1655 *avtA*: *panD*Bs-*ilvG*⁺*M,* Engineering Bacteria E. coli MG1655 *avtA*: *panDBl -ilvG*⁺*M* and Engineering Bacteria E. coli MG1655 *avtA: panDCg -ilvG⁺M,* in order to obtain engineering bacteria for VB5 production by fermentation. The VB5 yield of the engineering bacteria was compared by shake flask fermentation to verify the optimal PanD.

The fermentation method for producing VB5, the medium comprises a carbon source, a nitrogen source, inorganic ions, antibiotics and other nutritional factors. As the carbon source, sugars such as glucose, lactose, galactose, etc may be used. As an inorganic nitrogen source, an inorganic nitrogen source such as ammonia, ammonium sulfate, ammonium phosphate, ammonium chloride, etc can be used; and as an organic nitrogen source, an organic nitrogen source such as corn syrup, soybean meal hydrolysate, hair powder, yeast extract, peptone, etc can be used. The inorganic ions comprise one or more of iron, calcium, magnesium, manganese, molybdenum, cobalt, copper, potassium, and other ions.

Through the two-step validation of catalytic production of β-alanine and fermentation production of VB5, the L-aspartate α-decarboxylase with the highest activity was screened from 16 candidate enzymes from different origins with large sequence differences, and the VB5 can be efficiently produced by fermentation without the addition of β-alanine, which removes the bottleneck of biosynthesis and reduces the production cost.

The experimental methods in the following embodiments are conventional methods unless otherwise specified. The experimental materials used in the following embodiments were purchased from a conventional biochemical reagent store, unless otherwise specified. The quantitative tests in the following embodiments were set up for three repetitions, and the results were averaged. Unless otherwise specified, the technical means used in the following embodiments are conventional means well known to the skilled in the art and commercially available instruments and reagents, Please refer to the Experimental Guide to <Molecular Cloning (3rd Edition) > (Science Press), <Microbiology Experiment (4th Edition)> (Higher Education Press) as well as the manufacturer's manuals of the corresponding instruments and reagents, etc.

If the sequence in the specification is inconsistent with the sequence in the sequence list, the sequence in the specification will prevail.

The present application is further illustrated below in conjunction with the embodiments:

### Embodiment 1 Detection methods

The accumulation of β-alanine in the fermentation broth was quantitatively determined by HPLC, and the specific method is as follows. The supernatant of the fermentation broth was taken, diluted with purified water to the appropriate concentration, and filtered through 0.22µm filter membrane. The concentration of β-alanine was determined by on-line pre-column derivatization with o-phthalaldehyde (OPA). The chromatographic column used was Agilent AdvanceBio AAA C18 column (4.6×100 mm, 2.7 µm) , the column temperature was 40°C, the detection wavelength was 338 nm, and the flow rate of the mobile phase of 1 mL/min. The mobile phases of A were 10 mM Na₂HPO₄ and 10 mM Na₂B₄O₇, respectively, and the pH was adjusted to 8.2, and the mobile phase B was acetonitrile:methanol:water=45:45:10. β-alanine purchased from sigma was used as the standard, a standard curve of the concentration and the light absorption of alanine under the conditions of the chromatography was measured.

The yield of VB5 in the fermentation broth was quantitatively determined by HPLC as follows. The supernatant of the fermentation broth was diluted to the appropriate concentration with purified water and filtered through 0.22µm filter membrane. The chromatographic column used was Agilent ZORBAX SB-Aq column (4.6×250 mm), the column temperature was 30°C, the detection wavelength was 210 nm and the flow rate of the mobile phase of 1 mL/min. The mobile phase was 3.12 g/L NaH₂PO₄-2H₂O, and the pH was adjusted to 2.2 with phosphoric acid. Calcium pantothenate purchased from sigma company was used as the standard, and the standard curve of the concentration and the light absorption value of 0.1-0.5 g/L calcium pantothenate was determined.

### Embodiment 2 Construction of the vector overexpressing L-aspartate α-decarboxylase and whole cell catalyzed β-Alanine-producing Engineering Bacteria

Sixteen L-aspartate α-decarboxylase genes, *panD,* synthesized at Gene Synthesis company are derived from *Bacillus amyloliquefaciens* (as shown in SEQ ID No. 1), *Bacillus subtilis* (as shown in SEQ ID No. 2), *Bacillus licheniformis* (as shown in SEQ ID No. 3), *Chlorobium phaeobacteroides* (as shown in SEQ ID No. 4), *Corynebacterium efficiens* (as shown in SEQ ID No. 5), *Corynebacterium glutamicum* (as shown in SEQ ID No. 6), *Corynebacterium marinum* (as shown in SEQ ID No. 7), *Escherichia coli* (as shown in SEQ ID No. 8), *Haloquadratum walsbyi* (as shown in SEQ ID No. 9), *Hydrothermal vent metagenome* (as shown in SEQ ID No. 10), and *Methanocaldococcus jannaschii* (as shown in SEQ ID No. 11), *Magnetospirillum magneticum* (as shown in SEQ ID No. 12), *Metarhizium robertsii* (as shown in SEQ ID No. 13), *Mine drainage metagenome* (as shown in SEQ ID No. 14), *Rhodopirellula baltica* (as shown in SEQ ID No. 15), and *Thermotoga maritima* (as shown in SEQ ID No. 16). The XbaI and HindIII restriction endonuclease sequences were removed by synonymous codon substitutions during the above *panD* gene sequences was customized and synthesized.

In the present application, the above 16 BCD2-*panD* sequences were ligated to plasmids to construct 16 recombinant plasmids pET28a-BCD2-*panD*. When the above *panD* gene sequences was customized and synthesized, the same BCD2 sequences (as shown in SEQ ID No. 17) were synthesized simultaneously before each *panD* sequence , and at the same time, XbaI and HindIII restriction enzyme cleavage sites were added at both ends of the BCD2-*panD* sequences. The synthesized sequence was ligated into the vector. The above synthesized BCD2-*panD* vector and pET28a(+) plasmid were double digested using restriction endonucleases XbaI and HindIII, and the digested gene fragment of BCD2-*panD* and the linearized vector segment were recovered by gel electrophoresis, and the two fragments were further ligated using T4 ligase, and the ligated products were transformed into E. coli DH5α competent cells, and LB plates containing 50 mg/L kanamycin were used to screen the transformants containing the recombinant plasmid. Plasmids were extracted after amplicon amplification and sent for sequencing, and 16 correct plasmids were verified to be obtained pET28a-BCD2-*panDBa*,pET28a-BCD2-*panDBs*,pET28a-BCD2-*panDBl*,pET28a-BC D2-*panDCp*,pET28a-BCD2-*panDCe*,pET28a-BCD2-*panDCg*,pET28a-BCD2-*panDC m*,pET28a-BCD2-*panDEc*,pET28a-BCD2-*panDHw*,pET28a-BCD2-*panDHv*,pET28a -BCD2-*panDMj*,pET28a-BCD2-*panDMm*,pET28a-BCD2-*panDMr*,pET28a-BCD2-*p anDMd*,pET28a-BCD2-*panDRb*,pET28a-BCD2-*panDTm.* In the 16 recombinant vectors, 16 *panD* genes of different origins were used to regulate transcriptional initiation using the same promoter (T7) and to regulate translation initiation using the same BCD2 sequence.

The above extracted 16 pET28a-BCD2-*panD* plasmids expressing *panD* genes from different origins were transformed into E. coli BL21 (DE3) competent cells and screened on LB plates containing 50 mg/L kanamycin to obtain 16 strains of the engineering bacteria E. coli BL21 /pET28a-BCD2-*panD*Ba, E. coli BL21 /pET28a-BCD2-*panD*Bs, E. coli BL21 /pET28a-BCD2-*panDBl*, E. coli BL21 /pET28a-BCD2-*panD*Cp, E. coli BL21 /pET28a-BCD2-*panD*Ce, E. coli BL21 / pET28a-BCD2-*panDCg*, E. coli BL21 /pET28a-BCD2-*panDCm,* E. coli BL21 /pET28a-BCD2-*panDEc,* E. coli BL21 /pET28a-BCD2-*panDHw*, E. coli BL21 /pET28a- BCD2-*panDHv,* E. coli BL21 /pET28a-BCD2-panDMj, E. coli BL21 /pET28a-BCD2-*panDMm,* E. coli BL21 /pET28a-BCD2-*panDMr*, E. coli BL21 /pET28a-BCD2- *panDMd,* E. coli BL21 /pET28a-BCD2-*panDRb*, and E. coli BL21 /pET28a-BCD2-*panDTm,* for β-alanine whole-cell catalysis.

### Embodiment 3 Whole-cell catalytic screening for optimal panD genes

The above 16 engineering bacteria *E. coli BL21* /*pET28a-BCD2 panD* bacterial moss were scraped and inoculated into a 50 mL sterile breathable lid test tube containing 3 mL LB culture medium (containing 50 mg/L kanamycin), placed in a 37°C shaker at 220 rpm culture for 12 h, to obtain the seed liquid, with the OD₆₀₀ of 4-5; the seed liquid obtained from the cultivation was inoculated into a 500 mL baffled shake flask containing 30 mL LB medium (containing 50 mg/L kanamycin) at a 2% inoculum volume, placed in a 37°C shaker at 220 rpm culture for 2 h, and then added with 0.3 mM of IPTG, and continued to be induced for 4 h under the same conditions. 30 mL of aspartic acid solution was added to the baffled shake flask after induction (the pH was adjusted to 6.0 with sodium hydroxide), and the baffled shake flask was put in the 37°C shaker at 220 rpm for 30 min. After centrifugation, the supernatant was taken to measure the yield of β-alanine, and three parallel experiments were set up for each engineering bacterium and the average value was taken. The β-alanine yields of the engineering bacteria overexpressing *panD* genes from different orgins are shown in Table 1, and the β-alanine yields of the engineering bacteria overexpressing *panD* genes derived from *Bacillus subtilis* (as shown in SEQ ID No. 2), *Bacillus licheniformis* (as shown in SEQ ID No. 3), *C. glutamicum,* and *M. magneticum* is about ten times higher than that of other engineering bacteria, among which the L-aspartate α-decarboxylase from *Bacillus licheniformis* has the highest catalytic efficiency.

**Table 1**

| engineering bacteria | β-Alanine ( g/L ) |
|---|---|
| *E. coli* BL21 /pET28a-BCD2-*panDBa* | 0.22±0.02 |
| *E. coli* BL21 /pET28a-BCD2-*panDBs* | 3.45±0.44 |
| *E. coli* BL21 /pET28a-BCD2-*panDBl* | 4.12±0.35 |
| *E. coli* BL21 /pET28a-BCD2-*panDCp* | 0.05±0.01 |
| *E. coli* BL21 /pET28a-BCD2-*panDCe* | 0.35±0.05 |
| *E. coli* BL21 /pET28a-BCD2-*panDCg* | 2.98±0.42 |
| *E. coli* BL21 /pET28a-BCD2-*panDCm* | 0.44±0.02 |
| *E. coli* BL21 /pET28a-BCD2-*panDEc* | 0.55±0.11 |
| *E. coli* BL21 /pET28a-BCD2-*panDHw* | 0.35±0.02 |
| *E. coli* BL21 /pET28a-BCD2-*panDHv* | 0.05±0.01 |
| *E. coli* BL21 /pET28a-BCD2-*panDMj* | 0.58±0.06 |
| *E. coli* BL21 /pET28a-BCD2-*panDMm* | 1.21±0.11 |
| *E. coli* BL21 /pET28a-BCD2-*panDMr* | 0.61±0.03 |
| *E. coli* BL21 /pET28a-BCD2-*panDMd* | 0.02±0.01 |
| *E. coli* BL21 /pET28a-BCD2-*panDRb* | 0.21±0.02 |
| *E. coli* BL21 /pET28a-BCD2*-panDTm* | 0.74±0.10 |

The catalytic performance of the engineering bacteria *E. coli BL21* /*pET28a-BCD2-panDBl* was further verified using a 5L bioreactor. The moss of *E*. *coli BL21*/*pET28a-BCD2-panDBl* was scraped and inoculated into a 500 mL triangular flask containing 50 mL of LB medium(containing 50 mg/L kanamycin (5~200 mg/L is acceptable) ), and cultured in a 37°C shaker at 220 rpm for 4 h to obtain the seed liquid with OD₆₀₀ of 4-5; the seed liquid obtained from the culture was inoculated with 2% of the inoculum into a 5 L bioreactor containing 2 L of inorganic salts medium, the culture temperature is 37 °C, the DO is controlled above 30%, and the tank pressure is controlled at 0.02 ~ 0.10 MPa. The pH was maintained at 6.9 under the control of feedback supplement of ammonia, and the glucose concentration in the culture medium was maintained at 5 g / L or less by supplying glucose reservoir. When the OD₆₀₀ of the bacteria in the culture medium reached 30, 0.1 mM inducer IPTG was added, and the OD₆₀₀ of the bacteria in the culture medium reached about 80 after 4h. 10g/L of L-aspartic acid was added as a substrate, and the pH was not regulated during the catalytic process, and 10g/L of L-aspartic acid was added when the pH no longer increased. The solid aspartic acid was suppled 25 times intermittently, and the yield of β-alanine reached 140.5 g/L after 36 h of catalysis.

The inorganic salt medium components and the glucose reservoir components were as follows: inorganic salt medium: 2 g/L (NH₄)₂HPO₄, 4 g/L KH₂PO₄, 0.85 g/L Citric acid, 0.7 g/L MgSO₄·7H₂O, 10 mg/L FeSO₄·7H₂O, 2.25 mg/L ZnSO₄·7H₂O, 0.2 mg/L CuSO₄·5H₂O, 0.5 mg/L MnSO₄·5H₂O, 0.23 mg/L NaB₄O₇·10H₂O, 2.0 mg/L CaCl₂·2H₂O, 0.1 mg/L NH₄Mo₇O₂, 0.15 mg/L CoCl₂·6H₂O, and the remaining amount was water. The glucose reservoir contained 700 g/L glucose and 20 g/L MgSO4-7H2O, and the remaining amount was water.

### Embodiment 4 Construction of engineering bacteria for the production of VB5 by fermentation

The nucleotide sequence amplified by PCR using high-fidelity polymerase KAPA HiFiTM HotStar with P1 and P2 as primers and genomic DNA of wild-type E. coli strain K12 MG1655 as a template is shown in SEQ ID No. 18, in which 10nt-45nt is the promoter trc, 74nt-868nt is the coding sequence of *panB* gene, and 880nt-1731nt is the coding sequence of *panC* gene. The primer P1 was designed to introduce the strong promoter trc, and primers P1 and P2 were designed to introduce BamHI and SphI restriction endonuclease sites at the 5' end of primer P1 and P2, respectively. The PCR program was: denaturation at 98°C for 30 seconds, annealing at 65°C for 15 seconds, and extension at 72°C for 90 seconds, with 26 cycles, and a fragment of the *P_{trc}-panBC* gene of about 1800 bp was obtained.
P1 : 5'- CGCGGATCC
   *CAATTAATCATCCGGCTCGTATAATGTGTGG**A***GCACAACATCAATTTATCAGGA (as shown in SEQ ID No. 24; the underlined sequence is the BamHI cleavage recognition site, and the italicized sequence is the sequence of promoter trc)
P2 : 5'- ACATGCATGC CCTGTGTTAT GACAGATGAC -3'
   (as shown in SEQ ID No. 25; the underlined sequence is SphI cleavage recognition site)

The PCR amplified *P_{trc}-panBC* product was identified and recovered by gel electrophoresis, then, the Ptrc*-panBC* product and the pACYC184 plasmid was double digested with BamHI and SphI. The above PCR electrophoresis bands were recovered by gel cutting, and the DNA fragments of the amplified *P_{trc}-panBC* gene and pACYC184 plasmid were double digested with restriction endonucleases BamHI and SphI. The double-cleaved Ptrc-*panB*C and pACYC184 plasmids were recovered by gel electrophoresis, ligated with T4 ligase, and the ligation products were chemically transformed into E. coli DH5α competent cells, and chloramphenicol plates are coated after the transformed competent cells were resuscitated for 1 hour. The coated plates were placed in a 37°Cincubator for 12 hours, single colonies were picked for passaging, and the recombinant plasmid was extracted and sequenced to obtain the correct recombinant plasmid pACYC184-panBC.

The nucleotide sequence amplified by PCR with P3 and P4 as primers and genomic DNA of E. coli strain K12 MG1655 as a template is shown in SEQ ID No. 19, in which 11nt-45nt was the promoter of PJ23119, 66nt-977nt was the coding sequence of *panE* gene, and 988nt-1731nt was the terminator sequence. The promoter PJ23119 was designed on the amplification primer P3, the terminator L3S2P56 sequence was designed on the primer P4, and the SphI and BsaBI restriction endonuclease sites were designed at the 5' end of primers P3 and P4, respectively. Using the PCR reaction conditions mentioned above, the PJ23119-panE product obtained by amplification was identified and recovered by gel electrophoresis, and then was double digested using using SphI and BsaBI. The pACYC184-Ptrc-panBC plasmid were simultaneously double digested with SphI and BsaBI. The double digested PJ23119-panE and pACYC184-panBC plasmids were recovered by gel electrophoresis, ligated with T4 ligase,, and the ligated products were chemically transformed into Escherichia coli DH5α competent cells, which were resuscitated for 1 hr and coated on chloramphenicol plate. The coated plate was placed in a 37°C incubator for 12 hours, and single colonies were picked for passaging, and the recombinant plasmids were extracted and sequenced to obtain the correct recombinant plasmid, pACYC184-panBCE, thus obtaining the recombinant plasmid for overexpressing a vitamin B5 terminal synthesis pathway gene.
P3 : 5'- ACATGCATGC
   *ttgacagctagctcagtcctaggtataatgctagc*GTTGCGGGTGAGGAGGAACA
   (as shown in SEQ ID No. 26; the underlined sequence is the SphI restriction recognition site and the italicized sequence is the sequence of promoter J23119 )
P4 : 5'- CTCGATTTAGATC*CCAAAACGAA AAAAGACGCGCTTTTCAGC GTCTTTTTTC GAAAATT*AGT CTCTTCACTA CCAGGGATGA CTATCGAG
   (as shown in SEQ ID No. 27; the underlined sequence is the BsaBI restriction recognition site and the italic is the L3S2P56 terminator sequence)

Application of the reported CRISPR-Cas9 gene editing system including pCas9 and pTargetF vectors. ( Jiang, Y., Chen, B., Duan, C. L., Sun, B. B., Yang, J. J., and Yang, S. (2015) Multigene Editing in the Escherichia coli Genome via the CRISPR-Cas9 System, Appl Environ Microb 81, 2506-2514. ) .

The pTargetF vector was mutated using the Q5^{®} Site-Directed Mutagenesis Kit (Item No. E0552S) from NEB, with primers P5 and P6 designed according to the kit instructions. The mutated N20 sequence is CTTTTCCAAGC TGGGTCTACC, targeting the *avtA* gene. The mutated pTargetF is named pTargetFavtA.
P5: TGGGTCTACCG TTTTAGAGCT AGAAATAGC (as shown in SEQ ID No. 28);
P6: GCTTGGAAAG GACTAGTATT ATACCTAGG (as shown in SEQ ID No. 29);
P7:CG GACTGGAAGA AGATCTG (as shown in SEQ ID No.30);
P8:TTTCTTAGAC GTCGGAATTG AGACTCATGC ACAGCACGA (as shown in SEQ ID No.31);
P9:**TCGTGCTGT GCATGAGTCT**CAATTCC**GACGTC**TAAGAAAC
   (as shown in SEQ ID No. 32);
P10:GATCTCCTTT TTAAGTGAAC TTGGGGTCAG TGCGTCCTGC TGAT (as shown in SEQ ID No. 33);
P11: ATCAGCAGGACGCACTGACCCCAAGTTCACTTAAAAAGGAGAT C (as shown in SEQ ID No. 34);
P12:TGCCGTTCAT ATTGGTGATG CAAAAAACCC CTCAAGACC(as shown in SEQ ID No. 35);
P13:GGTCTTGAGGGGTTTTTT**GCATC ACCAATATGAACGGCA** (as shown in SEQ ID No. 36);
P14:GCTGATAGAG CTGCTTGGT (as shown in SEQ ID No. 37);
P15: **GGAGCTACTC ACACTGCTTG** (as shown in SEQ ID No. 38) ;
P16: CGCATACATT GATGCGTATG (as shown in SEQ ID No. 39) ;

The upstream sequence of the *avtA* gene was amplified using primers P7 and P8, the PL promoter was amplified using primers P9 and P10. The BCD2-*panDBs*-Ter, BCD2-*panDB*L-Ter, BCD2-*panDCg*-Ter gene fragments were amplified using primers P11 and P12, respectively, with pET28a-BCD2-*panDBs*, pET28 a-BCD2-*panDBl, E. coli* BL21/pET28 a-BCD2-*panDCg* as templates, and the downstream sequence of *avtA* gene was amplified using primers P13 and P14. The above four fragments were ligated by overlapping PCR to obtain an assemblage of the four DNA fragments DonorBs (as shown in SEQ ID No. 20), DonorBl (as shown in SEQ ID No. 21), and DonorCg (as shown in SEQ ID No. 22), which served as the templates for gene editing. Where 1nt-312nt of SEQ ID No.20, 21 and 22 are upstream sequences of the target gene *avtA* gene, 313nt-474nt are PL promoters, 475nt-560nt are BCD2 sequences. 560nt-943nt of SEQ ID No.20 are panDBs sequences, 944nt-995nt are terminator sequences, and 996-1261nt are sequences downstream of the *avtA* gene. 560nt-943nt of SEQ ID No. 21 are panDBl sequences. 944nt-995nt are terminator sequences, and 996-1261nt are sequences downstream of *avtA* gene. 560nt-970nt of SEQ ID No.22 are panDCg sequences, 971nt-1022nt are terminator sequences, and 1023-1288nt are sequences downstream of the *avtA* gene.

The pCas9 plasmid was transformed into MG1655, then, coated on kanamycin-resistant plates containing 50 mg/L, and incubated at 30°C to obtain strain MG655/pCas9. MG1655/pCas9 moss was picked in 50 mL of kanamycin-containing LB in 500 mL shake flasks, cultured at 30°C at 220 rpm, and when the OD₆₀₀ of the medium was 0.2, arabinose at the final concentration 10 mM was added for induction, and the competent cells were prepared when the medium OD₆₀₀ was 0.45. Two microliters of pTargetF*avtA* plasmid and 10 microliters of DonorBs template DNA were taken and electro-transformed into MG655/pCas9 competent cells, which were coated on the double-resistant plate containing 50 mg/L kanamycin and 50 mg/L spectacularin and incubated at 30°C. Single colonies integrating PL-BCD2-*panD*-Ter on the *avtA* gene were identified using primers P15 and P16, and the correctly sized PCR product was verified by sequencing. The correctly sequenced single colonies were selected and cultured with 0.2 mM IPTG to eliminate the pTargetFavtA plasmid to obtain the engineering bacteria *E. coli MG1655 avtA:panDBs*/*pCas, E. coli MG1655 avtA:panDBl*/*pCas, E. coli MG1655 avtA: panDCg pCas.* and still prepare competent cells and ready for use according to the above method.

Engineering bacteria *E. coli MG1655 avtA:panDBs*/*pCas, E.coli MG1655 avtA.panDBl*/*pCas and E. coli MG1655 avtA:panDCg*/*pCas* were inoculated into non-resistant LB liquid medium, cultured at 37 °C for 12 hours, then the liquid medium was diluted and coated on LB plates to obtain the engineering bacteria *E.coli* MG1655 *avtApanD*Bs, *E.coli* MG1655 *avtA:panDBl,* and *E.coli* MG1655 *avtA:panDCg* that eliminated the pCas plasmid, respectively. The gene *panD* was inserted into the coding sequence of the chromosomal *avtA* gene, which leads to the inactivation of *avtA* and weakens the valine competition metabolic pathway.

Wild-type Escherichia coli K12 MG1655 has a mutation in the *ilvG* gene, which encodes an inactive acetolactate synthase. In the present application, the active *ilvG* gene of E. coli BL21 was introduced on the chromosome of E. coli MG1655, which improved the synthesis of the precursor acetolactate of VB5. The *ilvG⁺M* gene derived from E. coli BL21 was inserted into the chromosome of E. coli K12 MG1655 and the trc strong promoter was used to regulate the transcription initiation of *ilvG*⁺*M* and the terminator Ter was used to regulate the transcription termination of *ilvG⁺M.* The *ilvG⁺M* gene was integrated into another N20 target sequence of the *avtA* gene. The pTargetF vector was mutated using the Q5^{®} Mutagenesis Kit and primers P17 and P18. pTargetF was named pTargetFavtA1 after the mutation.
P17: ACGGTCCACAG TTTTAGAGCT AGAAATAGC (as shown in SEQ ID No. 40);
P18: CGTAGTTACA GACTAGTATT ATACCTAGG (as shown in SEQ ID No. 41);
P19: GGCAGAAAAT CAGCCAGTTC (as shown in SEQ ID No. 42);
P20:TCCACACATT ATACGAGCCG GATGATTAAT TGTCAAGAAC TCTGTAGCAA GGAAGG (as shown in SEQ ID No. 43);
P21:TTGA CAATTAATCATCCGGCTCGTATAATGTGTGGA CAAGATT CAGGACGGGG AAC (as shown in SEQ ID No. 44);
P22:CGAAAAAAGA CGCTCTAAAA GCGTCTCTTT TCTGGTATAT TCCTTTTGCG CTCAG (as shown in SEQ ID No. 45) ;
P23:CAGAAAAGAGACGCTTTTAGAGCGTCTTTTTTCGTTTT**GGAGCTA CTC ACACTGCTTG** (as shown in SEQ ID No. 46) ;
P24:GCCAATATGC AGATGCTCA**TGAGCATCTGCATATTGG C** (as shown in SEQ ID No.47)
P25: CACGTTCGGA TATGAACTG (as shown in SEQ ID No. 48) ;
P26:CGTCAAGCTT CAGCAACTC (as shown in SEQ ID No. 49).

The upstream sequence of the *avtA* gene was amplified using primers P19 and P20, the *ilvG+M* sequence of E. coli BL21 was amplified using primers P21 and P22, and the downstream sequence of the *avtA* gene was amplified using primers P23 and P24. The trc promoter TTGA
CAATTAATCATCCGGCTCGTATAATGTGTGTGG**A** was introduced by primers P20 and P21, and the terminator sequence
CCAGAAAAGAGAGACGCTTTTAGAGCGTCTTTTTTTTCGTTTT was introduced by primers P22 and P23. The three fragments mentioned above were ligated using overlapping PCR. The assembled Donor *ilvGM* (as shown in SEQ ID No. 23) was obtained as a template for gene editing. 1-305nt of SEQ ID No. 23 is the upstream sequence of the *avtA* gene of the target gene, 306nt-341nt is the trc promoter, 367nt-2013nt is the *ilvG⁺* gene derived from *E.coli* BL21 coding sequence, 2010nt-2273nt is the coding sequence of *ilvM* gene, 2274-2328 is the terminator sequence, and 2329-2629 is the downstream sequence of *avtA* gene.

Two microliters of pTargetF*avtA*1 plasmid and 10 microliters of DonorilvGM template DNA were taken and respectively electro-transformed into *E.coli* MG1655 *avtA:panD*Bs/pCas, *E.coli* MG1655 *avtA:panDBl*/pCas, *E.coli* MG1655 *avtA:panDCg*/ pCas competent cells, then coated on the double-resistant plates containing 50 mg/L kanamycin and 50 mg/L spectacularin, and incubated at 30°C. Single colonies integrating Ptrc-ilvG⁺M-Ter on the *avtA* gene were identified using primers P25 and P26, and the PCR product with correct size was verified by sequencing. Single colonies with correct sequencing were selected and cultured with 0.2 mM IPTG to eliminate the pTargetFavtA1 plasmid. Non-resistant LB liquid medium was further added to, cultured at 37°C for 12 h, diluted and coated with LB plates, respectively, to obtain the engineering bacteria *E.coli MG1655 avtA:panDBs-ilvG⁺M, E. coli MG1655 avtA:panDBL-ilvG⁺M and E. coli MG1655 avtA:panDCg-ilvG⁺M* that eliminated the pCas plasmid. The synthesis of the VB5 precursor acetolactate was improved by the integration of active *ilvG⁺M* on the chromosome.

The above constructed vector pACYC184-*panB*CE was transformed into the above engineering bacteria *E.coli MG1655 avtA:panDBs-ilvG*⁺*M, E.coli MG1655 avtA:panDBl-ilvG*⁺*M,* and *E. coli MG1655 avtA:panDCg-ilvG*⁺*M* to obtain respectively engineering bacteria *E.coli MG1655 avtA:panDBs-ilvG*⁺*M*/*pACYC184-panBCE, E. coli MG1655 avtA:panDBl-ilvG*⁺*M*/*pACYC184-panBCE and E. coli MG1655 avtA.panDCg-ilvG*⁺*M*/*pACYC184-panBCE* for fermentation to produce VB5.

Among them:
**SEQ ID No.1 :** 
ATGATGGCCGGAAAACTGCACCGCGCTACCGTGACGGAAGCCAATTTAAA
CTATGTCGGC
AGCATAACGATTGATGAAGATCTTTTAGATGCCGTCGGAATGCTCGCTAATG
AAAAAGTT
CAGATTGTGAATAATAATAACGGAGCGAGACTTGAAACGTATATTATTCCCG
GTAAGCGG
GGGAGCGGCGTCATCTGTTTAAATGGAGCTGCCGCCCGTCTCGTCCAAGAA
GGAGATAAA
GTCATTATTATTTCTTATCAAATGATGTCTGATCAGGAAGCAAAAAGCCATC
AGCCGAAG
GTGGCCGTTCTGGATGATCAGAATAAAATCGAGCAGATGCTGGGCCAGGAG
CCGGCACAC
 ACGATTTTGTAA
**SEQ ID No.2** : 
ATGTATCGAA CAATGATGAG CGGCAAACTT CACAGGGCAA
CTGTTACGGA AGCAAACCTG
AACTATGTGG GAAGCATTAC AATTGATGAA GATCTCATTG ATGCTGTGGG
AATGCTTCCT
AATGAAAAAG TACAAATTGT GAATAATAAT AATGGAGCAC GTCTTGAAAC
GTATATTATT
CCTGGTAAAC GGGGAAGCGG CGTCATATGC TTAAACGGTG
CAGCCGCACG CCTTGTGCAG
GAAGGAGATA AGGTCATTAT TATTTCCTAC AAAATGATGT CTGATCAAGA
AGCGGCAAGC
CATGAGCCGA AAGTGGCTGT TCTGAATGAT CAAAACAAAA
TTGAACAAAT GCTGGGGAAC
GAACCAGCCC GTACAATTTT GTAA
**SEQ ID No.3** : 
ATGTACCGTA CGTTAATGAG CGCAAAACTT CACAGAGCGA
GAGTGACGGA AGCCAATTTG
AACTACGTCG GCAGCGTGAC AATTGATGAA GATTTGCTGG ATGCTGTCGG
AATGATGGCA
AATGAAAAAG TGCAAATTGT GAATAATAAT AACGGGGCCC
GGCTGGAAAC GTACATTATT
CCCGGTGAAA GGGGCAGCGG CGTCGTTTGT TTAAACGGAG
CTGCCGCCCG CCTTGTCCAG
GTTGGAGATG TCGTCATCAT CGTGTCTTAT GCGATGATGT CTGAAGAGGA
AGCAAAGACC
CATAAGCCGA AGGTTGCCGT TTTGAACGAG AGAAACGAAA
TCGAGGAAAT GCTGGGTCAG
GAGCCAGCCC GTACCATTCT GTAA
**SEQ ID No.4** : 
ATGAAGCTGCACCTGCTGAAGAGCAAAATTCACAACGCGCGTGTTACCAG
CGGTGACCTGGAGTACGAAGGCAGCATTACCATCGATCAGGAGCTGCTGCT
GCTGGCGGAGATGATCCCGAACGAAAAAGTTCTGGTGGTGAACAACAACA
ACGGCGAGCGTTTCGAAACCTATATCATTAACGGTGAACCGGGCAGCCGTG
TTATTCAGCTGAACGGTGCGGCGCGTTGCGCGCTGCCGGGCGACGAG
ATCATTATCATGACCTTCGCGGTGATGGATGAAAAGAAAGCGCGTACCTTTC
AACCGATGGTGCTGATCGTTGACCACCTGAACAACCCGAAGCGTCGTCAC
CGTATTGGCCAGGAAGACGAACAACTGAGCAGCAGCATCTAA
**SEQ ID No.5** : 
ATGCTGCGCACCATCCTCGGTAGCAAGATTCACCGCGCCACCGTCACCCAG
GCCGACCTT
GACTATGTCGGCTCCATCACCATCGACGCCGACCTGGTCAATGCCGCCGGC
CTCATCGAG
GGCGAGAAGGTCGCCGTCGTGGACATCACCAACGGCGCCCGCATTGAGAC
CTATGTGATC
ACCGGCGATGCCGGAACCGGCAGCATCTGCATCAATGGTGCCGCCGCCCAT
CTGATCAAC
CCGGGTGATCTGGTCATCATCATGAGCTATCTGCAGGCCACCGATGCCGAG
GCCCGCGCC
TACCAGCCCAATATCGTCCACGTGGATGCCGACAACCGGATCGTCGCCCTG
GGCAACGAC
GCCGGCGAGCCCATCCCGGGTTCCAGCTTGCTGTCCTCGCGCTCCCTCTAA
**SEQ ID No.6 :** 
ATGCTGCGCA CCATCCTCGG
AAGTAAGATT CACCGAGCCA CTGTCACTCA AGCTGATCTA GATTATGTTG
GCTCTGTAAC
CATCGACGCC GACCTGGTTC ACGCCGCCGG ATTGATCGAA
GGCGAAAAAG TTGCCATCGT
AGACATCACC AACGGCGCTC GTCTGGAAAC TTATGTCATT
GTGGGCGACG CCGGAACGGG
 
CAATATTTGC ATCAATGGTG CCGCTGCACA CCTTATTAAT CCTGGCGATC
TTGTGATCAT
CATGAGCTAC CTTCAGGCAA CTGATGCGGA AGCCAAGGCG
TATGAGCCAA AGATTGTGCA
CGTGGACGCC GACAACCGCA TCGTTGCGCT CGGCAACGAT
CTTGCGGAAG CACTACCTGG
ATCCGGGCTT TTGACGTCGA GAAGCATTTAA
**SEQ ID No.7 :** 
ATGCTCAGAACCATCCTCGGCAGCAAAATCCACCGCGCCACGGTCACCCA
GGCAGACCTC
AACTACGTGGGGTCCGTCACGGTCGACGCCGACCTGCTCGCGGCCGCCGG
CCTCATCGAG
GGCGAAAAGGTGGCCATCGTCGACGTCACCAACGGTGCCAGGCTGGAGAC
CTACGTCATC
ACCGGTCGCCCGGGCACCGGCGAGATCTGCATCAACGGTGCGGCGGCGCA
CCTGATCCAC
CCGGGCGACATCGTCATCCTCATCTCCTACCTGCAGGCCACCCTGGACGAG
GCCCTCGAA
TACGAGCCGCGCATCGTCCACGTCGACGAGAACAACCGCATCGTCGCCCT
GGGCAATGAC
ATCGCCGAGGCCGTCCCGGGCTCCGACACCGTCTCCGCCCGGAACATCTAA
**SEQ ID No.8 :** 
ATGATTCGCA CGATGCTGCA GGGCAAACTC CACCGCGTGA
AAGTGACTCA TGCGGACCTG
CACTATGAAG GTTCTTGCGC CATTGACCAG GATTTTCTTG ACGCAGCCGG
TATTCTCGAA
AACGAAGCCA TTGATATCTG GAATGTCACC AACGGCAAGC GTTTCTCCAC
TTATGCCATC
GCGGCAGAAC GCGGTTCGAG AATTATTTCT GTTAACGGTG
CGGCGGCCCA CTGCGCCAGT
 
GTCGGCGATA TTGTCATCAT CGCCAGCTTC GTTACCATGC CAGATGAAGA
AGCTCGCACC
TGGCGACCCA ACGTCGCCTA TTTTGAAGGC GACAATGAAA
TGAAACGTAC CGCGAAAGCG
ATTCCGGTAC AGGTTGCTTAA
**SEQ ID No.9 :** 
ATGCGTCGTTGGCTGCTGAAGAGCAAACTGCACCGTGCGCGTGTGACCGG
TACCGAGAAGGACTACGAAGGCAGCATCAGCATTGATGCGGCGCTGCTGA
GCGAGGCGGACATTGCGGTGGGTGAACAGGTTCAAGTGGTTAACGTTACC
AACGGCGAGCGTTTCGAAACCTATACCATTGAGGGTGAAAGCCGTCAGATG
GAGCTGAACGGTGCGGCGCGTCTGGCGGAAACCGGTGATGTGATCAT
TGTTATCAGCTACGGCCTGTATGTGAAAGATGAGCAACCGGAACCGACCGT
TCTGCTGCTGGACGAGGAAAACCGTATTAGCGAGCGTGAATAA
**SEQ ID No.10 :** 
ATGCAGCGTACCTTCCTGAAGAGCAAACTGCACCGTCTGACCACCACCATC
CGTGGCAAGGAGAACAGCGGCGTGATTCTGGTTAACGGTGTGGCGCCGCA
CAAAGTTGCGGGCGACCTGCTGATCATTGCGGCGTACAGCAGCTATAGCGA
GGATGAACTGCGTAACTACCAACCGGCGCTGTGCTATGTTGACGAAAAGA
ACGTGCTGACCCGTATCAGCCGTTAA
**SEQ ID No.11** : 
ATGCGTAACATGCAGGAGAAAGGCGTGAGCGAGAAGGAAATTCTGGAGGA
ACTGAAGAAATACCGTAGCCTGGATCTGAAATATGAAGACGGCAACATTTT
CGGTAGCATGTGCAGCAACGTGCTGCCGATCACCCGTAAGATCGTTGATATT
TTCCTGGAGACCAACCTGGGTGACCCGGGCCTGTTTAAAGGTACCAAGCT
GCTGGAGGAAAAAGCGGTGGCGCTGCTGGGCAGCCTGCTGAACAACAAG
GATGCGTACGGTCACATCGTTAGCGGTGGCACCGAAGCGAACCTGATGGCG
CTGCGTTGCATCAAAAACATTTGGCGTGAGAAACGTCGTAAGGGCCTGAG
CAAAAACGAACACCCGAAGATCATTGTGCCGATTACCGCGCACTTCAGCTT
TGAGAAAGGTCGTGAAATGATGGATCTGGAGTACATCTATGCGCCGATTAA
AGAGGACTACACCATCGACGAAAAATTTGTGAAGGACGCGGTTGAGGACT
 
ATGATGTTGACGGTATCATTGGCATTGCGGGTACCACCGAACTGGGTACCAT
CGATAACATTGAGGAACTGAGCAAAATCGCGAAGGAAAACAACATCTACA
TTCACGTGGACGCGGCGTTCGGTGGCCTGGTTATCCCGTTTCTGGACGATA
AATACAAGAAAAAGGGCGTGAACTATAAGTTCGATTTTAGCCTGGGTGTTG
ATAGCATCACCATTGACCCGCACAAAATGGGTCACTGCCCGATCCCGAGCG
GTGGCATTCTGTTCAAAGACATCGGTTACAAGCGTTATCTGGATGTGGACG
CGCCGTATCTGACCGAGACCCGTCAGGCGACCATTCTGGGCACCCGTGTGG
GTTTTGGTGGCGCGTGCACCTACGCGGTTCTGCGTTATCTGGGCCGTGAAG
GTCAACGTAAAATCGTTAACGAGTGCATGGAAAACACCCTGTACCTGTATA
AAAAGCTGAAAGAGAACAACTTCAAGCCGGTGATCGAACCGATTCTGAAC
ATCGTTGCGATTGAGGATGAAGACTACAAAGAGGTGTGCAAAAAGCTGCG
TGACCGTGGTATTTATGTGAGCGTTTGCAACTGCGTTAAAGCGCTGCGTATC
GTGGTTATGCCGCACATTAAGCGTGAGCACATCGATAACTTTATCGAAATTC
TGAACAGCATCAAACGTGACTAA
**SEQ ID No.12 :** 
ATGATGAAGATCATTCGTGCGAAACTGCACGGCATCCGTGTGACCAACGCG
GATCTGAACTACCACGGCAGCATTACCCTGGACCCGGAGCAGTGCGAAATG
GCGGGTATCTATCCGATGGAGTTCGTTGATATTTGGAACAAGAACAGCGCG
GCGCGTATCAGCACCTACGTGATTTTTGGTGAACCGGGCAGCCGTTGCTGC
GTTCTGAACGGTGCGGCGCGTACCTGCCAAAAAGGCGACGAGCTGAT
CATTGCGGCGAGCGCGGATATCAGCGGTCCGGAAAAGCTGTACGACATCAA
ACCGCGTATTCTGACCTTCCTGCCGGACAACCACGTGGATCAGGTTCTGTA
CTATGATGTGTTCCAAAGCGAGAAGCGTCCGTATGACTTTCGTATCGTTGAC
GCGGATAAACACACCATTGAAAGCTGCCACACCTGGCCGAACGTGGACAT
CACCAAGCTGCGTAGCGATCTGGCGGCGAAAGGTTGGAGCGAGGCGGAAA
TCGACAGCTTCATTGCGAGCCACTTTAGCCTGTAA
**SEQ ID No.13** : 
ATGCGTGACACCAAGAAAATGCTGAACCGTGCGGATGAGCTGGACGATCT
GTACGAAGCGGTTCGTGCGCTGATCATTCCGCATGTGCGTGCGGCGGATGA
AGCGTGCAGCCTGAAGAGCGCGGGTCAGCTGCACACCGACGATACCCAGC
 
GTCTGCAAAACGTTCTGGTGGAACCGTACCCGCCGAAGGCGCTGCAGGAG
CGTTTCCAATTTACCCTGCCGGACAACGAGGGTAACGGCAAAGATGGCCTG
ATGCACCTGATCCGTGATGTTCTGCGTTATAGCGTGAACACCTGGGACCAA
GGTTTTATGGATAAGCTGACCAGCACCAACCCGGTTGGCGTGATCAGC
GAAATTGTTCTGGGCATCCTGAACACCAACGTTCATGTGTACCATGTGGCG
CCGGCGCTGAGCGTTATTGAGAAAGTGACCGGCCGTACCCTGGCGGCGTAT
TTCGGTTTTAACAGCCCGAGCGCGGGTGGCATTAGCTGCCAGGGTGGCAGC
GCGAGCAACCTGACCAGCCTGGTGGTTGCGCGTAACAGCCTGTACCCGGA
CTGCAAGCTGAACGGTGGCAGCAGCTATCAATTTGCGATCTTCACCAGCTG
CCACGGTCACTTCAGCATGGAAAAAGCGGCGATTACCTGCGGTATGGGCCT
GAGCAGCGTGGTTCACGTTCCGGTGAACGACGATGGCCGTATGAACGTTAG
CGCGCTGCGTGAGCTGGTGATCCAGGCGAAGGCGCAAGGTAAAACCCCGC
TGTATGTTAACGCGACCGCGGGTACCACCGTTCTGGGTGTGTTTGACCCGC
TGCACGAAATCAAGACCATTTGCGAGGAATTTGGCATGTGGTTCCACGTGG
ATGCGAGCTGGGGTGGCAGCATCATTTTCAGCGCGAAGCACCGTCACAAA
CTGACCGGTTGCGAGCTGGCGGACAGCCTGACCATTAGCCCGCACAAAAT
GCTGAACGTTCCGATGACCTGCAGCTTTCTGCTGACCAACAACCTGAGCAG
CTTCTACACCGCGAACAGCCTGGACGCGGGTTACCTGTTCCACGATACCGA
GGACGATGAAGTGTGGGACCTGGCGAACCTGACCCTGCAGTGCGGTCGTC
GTGCGGATAGCCTGAAAATGGCGCTGGCGTGGACCTACTATGGTGCGGCGG
GCTTTGAACGTCGTATCAACCACGCGTTCAAGATGGCGGCGCACCTGAGCA
GCATCATTCAGAAAAGCCCGGACTTTGAACTGGTTAGCCCGAACCCGCCGC
CGTGCCTGCAAGTGTGCTTCTACTATACCCCGGGTGGCAAGATGGCGAAAA
GCGAGATGGAAACCAGCCGTCGTACCCGTGCGATGGTTGAAAAGATGGTG
GACCGTGGCTTCATGTTTGATTTCGCGCCGGGTCCGAAAGGCGATTTCTTTC
GTGTGGTTGTGAACTGCGAGACCCTGCTGGGTACCGTTGAAGGCCTGTTCA
AGGGTCTGGAGGCGGTGGGTAAACAAGTTGTGTAA
**SEQ ID No.14 :** 
ATGCTGAACATCACCACCGGTGCGCGTTTCACCACCTACGCGATCGAAGCG
CCGCGTGGCAGCAAAGTGATTGGTGTGAACGGTGCGGCGCGTCTGGT
 
GCAGAAGGGTGACAAAGTTATCGTGGTTACCTACGGCATGCTGCCGGAGG
AAGAGGCGCGTAACTATAACCCGACCGTGGTTCTGCTGGACGATGGTAACC
TGATTAAACGTGCGGCGTAA
**SEQ ID No.15 :** 
ATGGTGGACACCCCGTACCGTAAGATGCTGGCGGCGAAAATCCACCGTGCG
ACCGTGACCGGTGCGGATGTTAACTATGAAGGCAGCCTGACCGTGCCGCCG
GAACTGCTGGTTGCGGCGAAGATCCACCCGTACGAGAGCCTGCACGTGTG
GAACGTTACCCGTGGTACCCGTCTGGAGACCTATGCGATTGAAGGCCTGCC
GAACAGCAACGACGTGTGCGCGAACGGTGCGGCGCACCTGATTCGTC
CGGGTGACCATGTGATTCTGGCGGCGTATGCGATGGTTCCGGAGGCGGATG
CGGCGACCCACAAACCGCGTCTGATCTTCGTGGACGATAACAACCAGCTG
AGCCACGTTGGTCCGGAAATTGCGGGTCCGAACCTGCGTAGCGACAGCGA
CGATACCCACCTGGTTCGTAGCACCGAGATGACCCCGGATGGTCAACCGCT
GGCGGAAGGCTGCTAA
**SEQ ID No.16** : 
ATGCTGAACATCTACCTGAAGAGCAAAATCCACATGGCGACCATTACCCGT
AAAGAGGTGTACTATGAAGGTAGCATCGAGATTGACGAGGAACTGATGGGT
AAAGCGGGCATTAGCGAGGGCGAACTGGTTCTGGTGGTGAACGTTAACAA
CGCGGAACGTTTCGTGACCTACGTTATCAAGGGTAAACGTGGCAGCCGTGA
GATTAACCTGTATGGTGCGGCGGCGCGTCTGGGCGAGGAAGGCGACCGTG
TGATCATTATGGCGTTCACCTTTAGCGATAAGCCGGTGAAGGCGAAAACCA
TCGTTCTGAACGAGAAGAACGAAATTGTTCAGGAAAAATAA
SEQ ID No.17 : 
CCAAGTTCACTTAAAAAGGAGATCAACAATGAAAGCAATTTTCGTACTGAA
ACATCTTAATCATGCTAAGGAGGTTTTCTAATG
**SEQ ID No.18** : 
CGCGGATCC TTGA CAATTAATCATCCGGCTCGTATAATGTGTGGA
GCACAACA
TCAATTTATCAGGATACGTTATGAAACCGACCACCATCTCCTTACTGCAGAA
GTACAAAC
 
AGGAAAAAAAACGTTTCGCGACCATCACCGCTTATGACTATAGCTTCGCCA
AACTCTTTG
CTGATGAAGGGCTTAACGTCATGCTGGTGGGCGATTCGCTGGGCATGACGG
TTCAGGGGC
ACGACTCCACCCTGCCAGTTACCGTTGCCGATATCGCCTACCACACTGCCG
CCGTACGTC
GCGGCGCACCAAACTGCCTGCTGCTGGCTGACCTGCCGTTTATGGCGTATG
CCACGCCGG
AACAAGCCTTCGAAAACGCCGCAACGGTTATGCGTGCCGGTGCTAACATG
GTCAAAATTG
AAGGCGGTGAGTGGCTGGTAGAAACCGTACAAATGCTGACCGAACGTGCC
GTTCCTGTAT
GTGGTCACTTAGGTTTAACACCACAGTCAGTGAATATTTTCGGTGGCTACA
AAGTTCAGG
GGCGCGGCGATGAAGCGGGCGATCAACTGCTCAGCGATGCATTAGCCTTAG
AAGCTGCTG
GGGCACAGCTGCTGGTGCTGGAATGCGTGCCGGTTGAACTGGCAAAACGT
ATTACCGAAG
CACTGGCGATCCCGGTTATTGGCATTGGCGCAGGCAACGTCACTGACGGGC
AGATCCTCG
TGATGCACGACGCCTTTGGTATTACCGGCGGTCACATTCCTAAATTCGCTAA
AAATTTCC
TCGCCGAAACGGGCGACATCCGCGCGGCTGTGCGGCAGTATATGGCTGAA
GTGGAGTCCG
GCGTTTATCCGGGCGAAGAACACAGTTTCCATTAAGGAGTCACGTTGTGTT
AATTA
TCGAAACCCTGCCGCTGCTGCGTCAGCAAATTCGCCGCCTGCGTATGGAAG
GCAAGCGCG
TGGCGCTGGTGCCTACCATGGGTAACCTGCACGATGGCCATATGAAGCTGG
TCGACGAAG
 
CCAAAGCCCGCGCCGATGTGGTCGTCGTCAGTATTTTCGTTAACCCGATGC
AGTTCGACC
GCCCGGAAGATCTGGCTCGTTATCCACGGACCTTGCAGGAGGACTGCGAG
AAGCTAAACA
AACGTAAAGTGGATTTAGTTTTCGCCCCTTCGGTAAAAGAGATCTACCCGA
ACGGTACTG
AAACCCACACTTACGTTGACGTTCCTGGCCTTTCGACCATGCTGGAAGGTG
CCAGCCGTC
CGGGACATTTTCGCGGCGTTTCGACTATTGTCAGCAAGCTGTTCAACCTGG
TCCAGCCGG
ACATCGCCTGCTTCGGTGAAAAAGATTTTCAGCAACTGGCGCTGATCCGCA
AAATGGTTG
CCGATATGGGCTTCGATATTGAGATTGTCGGTGTGCCAATTATGCGCGCCAA
AGACGGTC
TGGCGCTAAGTTCCCGTAACGGTTATCTGACGGCGGAACAACGCAAAATTG
CGCCTGGTC
TGTACAAAGTTTTAAGTTCGATTGCTGACAAATTGCAGGCTGGGGAACGGG
ATCTCGATG
AAATTATTACCATTGCGGGGCAAGAACTGAATGAAAAAGGCTTCCGCGCCG
ATGATATTC
AGATTCGCGATGCCGACACATTGCTGGAAGTTTCTGAAACCAGCAAACGG
GCAGTAATTC
TGGTAGCCGCCTGGCTTGGCGATGCTCGCCTGATCGACAACAAAATGGTCG
AGCTGGCGT
AATACTTAACTGGCGCTACGGCTGATGGCGCCAGTTATTAATTTACCCCACG
TCATCTGT
CATAACACAGG
**SEQ ID No.19 :** 
ACATGCATGC ttgacagctagctcagtcctaggtataatgctagc

GTTGCGGGTGAGGAGGAACAATGAAAATTACCGTATTGGGATGCGGTGCCT
 
TAGGGCAAT
TATGGCTTACAGCACTTTGCAAACAGGGTCATGAAGTTCAGGGCTGGCTGC
GCGTACCGC
AACCTTATTGTAGCGTGAATCTGGTTGAGACAGATGGTTCGATATTTAACGA
ATCGCTGA
CCGCCAACGATCCCGATTTTCTCGCCACCAGCGATCTGCTCCTGGTGACGC
TGAAAGCAT
GGCAGGTTTCCGATGCCGTCAAAAGCCTCGCGTCCACACTGCCTGTAACTA
CGCCAATAC
TGTTAATTCACAACGGCATGGGCACCATCGAAGAGTTGCAAAACATTCAGC
AGCCATTAC
TGATGGGCACCACCACCCATGCAGCCCGCCGCGACGGCAATGTCATTATTC
ATGTGGCAA
ACGGTATCACGCATATTGGCCCGGCACGGCAACAGGACGGGGATTACAGTT
ATCTGGCGG
ATATTTTGCAAACCGTGTTGCCTGACGTTGCCTGGCATAACAATATTCGCGC
CGAGCTGT
GGCGCAAGCTGGCAGTCAACTGCGTGATTAATCCACTGACTGCCATCTGGA
ATTGCCCGA
ACGGTGAATTACGTCATCATCCGCAAGAAATTATGCAGATATGCGAAGAAG
TCGCGGCGG
TGATCGAACGCGAAGGGCATCATACTTCAGCAGAAGATTTGCGTGATTACG
TGATGCAGG
TGATTGATGCCACAGCGGAAAATATCTCGTCGATGTTGCAGGATATCCGCGC
GCTGCGCC
ACACTGAAATCGACTATATCAATGGTTTTCTCTTACGCCGCGCCCGCGCGCA
TGGGATTG
CCGTACCGGAAAACACCCGCCTGTTTGAAATGGTAAAAAGAAAGGAGAGT
GAATATGAGC
GCATCGGCACTGGTTTGCCTCGCCCCTGGTAGTGAAGAGACTAATTTTCGA
 AAAAAGACGCTGAAAAGCGTCTTTTTTCGTTTTGGGATCTAAATCGAG
**SEQ ID No.20** : 
CG GACTGGAAGA AGATCTGTTT GTCTCTGCGC
GTCCGAATAT TGAACTGCTG CCGGAAGGCC AGTTTAAATA CCACGTCGAT
TTTGAGCATC
TGCATATTGG CGAAGAAACC GGGATGATTT GCGTCTCCCG
GCCGACGAAT CCAACAGGCA
ATGTGATTAC TGACGAAGAG TTGCTGAAGC TTGACGCGCT
GGCGAATCAA CACGGCATTC
CGCTGGTGAT TGATAACGCT TATGGCGTCC CGTTCCCGGG TATCATCTTC
AGTGAAGCGC
GCCCGCTATG GAATCCGAAT ATCGTGCTGT GCATGAGTCT
CAATTCCGACGTCTAAGAAACCATT
ATTATCATGACATTAACCTATAAAAA
TAGGCGTATCACGAGGCCCTTTCGTC
TTCACCTCGAGTCCCTATCAGTGATA
GAGATTGACATCCCTATCAGTGATAG
AGATACTGAGCACATCAGCAGGACG
CACTGACC
GGGCCCAAGTTCACTTAAAAAGGAGATCAACAATGAAAGCAATTTTCGTAC
TGAAACATCTTAATCATGCTAAGGAGGTTTTCTA
ATGTATCGAA CAATGATGAG CGGCAAACTT CACAGGGCAA
CTGTTACGGA AGCAAACCTG
AACTATGTGG GAAGCATTAC AATTGATGAA GATCTCATTG ATGCTGTGGG
AATGCTTCCT
AATGAAAAAG TACAAATTGT GAATAATAAT AATGGAGCAC GTCTTGAAAC
GTATATTATT
CCTGGTAAAC GGGGAAGCGG CGTCATATGC TTAAACGGTG
CAGCCGCACG CCTTGTGCAG
GAAGGAGATA AGGTCATTAT TATTTCCTAC AAAATGATGT CTGATCAAGA
 
AGCGGCAAGC
CATGAGCCGA AAGTGGCTGT TCTGAATGAT CAAAACAAAA
TTGAACAAAT GCTGGGGAAC
GAACCAGCCC GTACAATTTT GTAA
AAGC
CTAGCATAACCCCTTGGGGCCTCTAAACGGGTCTTGAGGGGTTTTTTG
CATC ACCAATATGAACGGCA
TTAT CAGCCTGGCA CCTGGCGGTA TTGGTCCGGC GATGATGTGT
GAAATGATTA
AGCGTAACGA TCTGCTGCGC CTGTCTGAAA CAGTCATCAA ACCGTTTTAC
TACCAGCGTG
TTCAGGAAAC TATCGCCATC ATTCGCCGCT ATTTACCGGA AAATCGCTGC
CTGATTCATA
AACCGGAAGG AGCCATTTTC CTCTGGCTAT GGTTTAAGGA TTTGCCCATT
ACGACCAAGC
AGCTCTATCA GC
**SEQ ID No.21 :** 
CG GACTGGAAGA AGATCTGTTT GTCTCTGCGC
GTCCGAATAT TGAACTGCTG CCGGAAGGCC AGTTTAAATA CCACGTCGAT
TTTGAGCATC
TGCATATTGG CGAAGAAACC GGGATGATTT GCGTCTCCCG
GCCGACGAAT CCAACAGGCA
ATGTGATTAC TGACGAAGAG TTGCTGAAGC TTGACGCGCT
GGCGAATCAA CACGGCATTC
CGCTGGTGAT TGATAACGCT TATGGCGTCC CGTTCCCGGG TATCATCTTC
AGTGAAGCGC
GCCCGCTATG GAATCCGAAT ATCGTGCTGT GCATGAGTCT
CAATTCCGACGTCTAAGAAACCATT
ATTATCATGACATTAACCTATAAAAA
TAGGCGTATCACGAGGCCCTTTCGTC
 
TTCACCTCGAGTCCCTATCAGTGATA
GAGATTGACATCCCTATCAGTGATAG
AGATACTGAGCACATCAGCAGGACG
CACTGACC
GGGCCCAAGTTCACTTAAAAAGGAGATCAACAATGAAAGCAATTTTCGTAC
TGAAACATCTTAATCATGCTAAGGAGGTTTTCTA
ATGTACCGTA CGTTAATGAG CGCAAAACTT CACAGAGCGA
GAGTGACGGA AGCCAATTTG
AACTACGTCG GCAGCGTGAC AATTGATGAA GATTTGCTGG ATGCTGTCGG
AATGATGGCA
AATGAAAAAG TGCAAATTGT GAATAATAAT AACGGGGCCC
GGCTGGAAAC GTACATTATT
CCCGGTGAAA GGGGCAGCGG CGTCGTTTGT TTAAACGGAG
CTGCCGCCCG CCTTGTCCAG
GTTGGAGATG TCGTCATCAT CGTGTCTTAT GCGATGATGT CTGAAGAGGA
AGCAAAGACC
CATAAGCCGA AGGTTGCCGT TTTGAACGAG AGAAACGAAA
TCGAGGAAAT GCTGGGTCAG
GAGCCAGCCC GTACCATTCT GTAA
AAGC
CTAGCATAACCCCTTGGGGCCTCTAAACGGGTCTTGAGGGGTTTTTTG
CATC ACCAATATGAACGGCA
TTAT CAGCCTGGCA CCTGGCGGTA TTGGTCCGGC GATGATGTGT
GAAATGATTA
AGCGTAACGA TCTGCTGCGC CTGTCTGAAA CAGTCATCAA ACCGTTTTAC
TACCAGCGTG
TTCAGGAAAC TATCGCCATC ATTCGCCGCT ATTTACCGGA AAATCGCTGC
CTGATTCATA
AACCGGAAGG AGCCATTTTC CTCTGGCTAT GGTTTAAGGA TTTGCCCATT
ACGACCAAGC
 AGCTCTATCA GC
**SEQ ID No.22 :** 
CG GACTGGAAGA AGATCTGTTT GTCTCTGCG C
GTCCGAATAT TGAACTGCTG CCGGAAGGCC AGTTTAAATA CCACGTCGAT
TTTGAGCATC
TGCATATTGG CGAAGAAACC GGGATGATTT GCGTCTCCCG
GCCGACGAAT CCAACAGGCA
ATGTGATTAC TGACGAAGAG TTGCTGAAGC TTGACGCGCT
GGCGAATCAA CACGGCATTC
CGCTGGTGAT TGATAACGCT TATGGCGTCC CGTTCCCGGG TATCATCTTC
AGTGAAGCGC
GCCCGCTATG GAATCCGAAT ATCGTGCTGT GCATGAGTCT
CAATTCCGACGTCTAAGAAACCATT
ATTATCATGACATTAACCTATAAAAA
TAGGCGTATCACGAGGCCCTTTCGTC
TTCACCTCGAGTCCCTATCAGTGATA
GAGATTGACATCCCTATCAGTGATAG
AGATACTGAGCACATCAGCAGGACG
CACTGACC
GGGCCCAAGTTCACTTAAAAAGGAGATCAACAATGAAAGCAATTTTCGTAC
TGAAACATCTTAATCATGCTAAGGAGGTTTTCTA
ATGCTGCGCA CCATCCTCGG
AAGTAAGATT CACCGAGCCA CTGTCACTCA AGCTGATCTA GATTATGTTG
GCTCTGTAAC
CATCGACGCC GACCTGGTTC ACGCCGCCGG ATTGATCGAA
GGCGAAAAAG TTGCCATCGT
AGACATCACC AACGGCGCTC GTCTGGAAAC TTATGTCATT
GTGGGCGACG CCGGAACGGG
CAATATTTGC ATCAATGGTG CCGCTGCACA CCTTATTAAT CCTGGCGATC
TTGTGATCAT
 
CATGAGCTAC CTTCAGGCAA CTGATGCGGA AGCCAAGGCG
TATGAGCCAA AGATTGTGCA
CGTGGACGCC GACAACCGCA TCGTTGCGCT CGGCAACGAT
CTTGCGGAAG CACTACCTGG
ATCCGGGCTT TTGACGTCGA GAAGCATTTAA
AAGC
CTAGCATAACCCCTTGGGGCCTCTAAACGGGTCTTGAGGGGTTTTTTG
CATC ACCAATATGAACGGCA
TTAT CAGCCTGGCA CCTGGCGGTA TTGGTCCGGC GATGATGTGT
GAAATGATTA
AGCGTAACGA TCTGCTGCGC CTGTCTGAAA CAGTCATCAA ACCGTTTTAC
TACCAGCGTG
TTCAGGAAAC TATCGCCATC ATTCGCCGCT ATTTACCGGA AAATCGCTGC
CTGATTCATA
AACCGGAAGG AGCCATTTTC CTCTGGCTAT GGTTTAAGGA TTTGCCCATT
ACGACCAAGC
AGCTCTATCA GC
**SEQ ID No.23 :**

GGCAGAAAAT CAGCCAGTTC CGCGCCCGCC ATCCCGCAAT
TGGCGCGGGC AAACAAACGA
CACTTTTGCT GAAGCAGGGC TACGGCTTTG TTCGTGAGCA TGGCGACGAT
AAAGTGCTGG
TCGTCTGGGC AGGGCAACAG TAACTTTTCC GGCTTCCCGT TCGTCAGTAC
CTCGGGAAGC
CGCCAACCAG GATAAAATGT CAGCCCTAAT CAGCGTTGCA
GGATAAAGCA CCGCTCACTC
TTCAACAGAC CGATTTGCAC CCCAGCAAAT GTAGCGTTAT TGTTACCTTC
CTTGCTACAG
AGTTCTTGA CAATTAATCATCCGGCTCGTATAATGTGTGGA
CAAGATT CAGGACGGGG AACTAACTAT G

AATGGCGCACAGTGGGTGGTACATGCGTTGCGGGCACAGGGTGTGAACAC
CGTTTTCGGTTATCCGGGTGGCGCAATTATGCCGGTTTACGATGCATTGTATG
ACGGCGGCGTGGAGCACTTGCTATGCCGACATGAGCAGGGTGCGGCAATG
GCGGCTATCGGTTATGCTCGTGCTACCGGCAAAACTGGCGTATGTATCGCCA
CGTCTGGTCCGGGCGCAACCAACCTGATAACCGGGCTTGCGGACGCACTG
TTAGATTCCATCCCTGTTGTTGCCATCACCGGTCAAGTGTCCGCACCGTTTA
TCGGCACTGACGCATTTCAGGAAGTGGATGTCCTGGGATTGTCGTTAGCCT
GTACCAAGCACAGCTTTCTGGTGCAGTCGCTGGAAGAGTTGCCGCGCATCA
TGGCTGAAGCATTCGACGTTGCCTGCTCAGGTCGTCCTGGTCCGGTTCTGG
TCGATATCCCAAAAGATATCCAGTTAGCCAGCGGTGACCTGGAACCGTGGT
TCACCACCGTTGAAAACGAAGTGACTTTCCCACATGCCGAAGTTGAGCAA
GCGCGCCAGATGCTGGCAAAAGCGCAAAAACCGATGCTGTACGTTGGCGG
TGGCGTGGGTATGGCGCAGGCAGTTCCGGCTTTGCGTGAATTTCTCGCTGC
CACAAAAATGCCTGCCACCTGTACGCTGAAAGGGCTGGGCGCAGTAGAAG
CAGATTATCCGTACTATCTGGGCATGCTGGGAATGCATGGCACCAAAGCGG
CGAACTTCGCGGTGCAGGAGTGCGACTTGCTGATCGCCGTGGGTGCACGT
TTTGATGACCGGGTGACCGGCAAACTGAACACCTTCGCACCACACGCCAG
TGTTATCCATATGGATATCGACCCGGCAGAAATGAACAAGCTGCGTCAGGC
ACATGTGGCATTACAAGGTGATTTAAATGCTCTGTTACCAGCATTACAGCAG
CCGTTAAATATCAATGACTGGCAGCTACACTGCGCGCAGCTGCGTGATGAA
CATGCCTGGCGTTACGACCATCCCGGTGACGCTATCTACGCGCCGTTGTTGT
TAAAACAACTGTCAGATCGTAAACCTGCGGATTGCGTCGTGACCACAGATG
TGGGGCAGCACCAGATGTGGGCTGCGCAGCACATCGCCCACACTCGCCCG
GAAAATTTCATCACCTCCAGCGGCTTAGGCACCATGGGTTTTGGTTTACCG
GCGGCGGTTGGCGCGCAAGTCGCGCGACCAAACGATACCGTCGTCTGTATC
TCCGGTGACGGCTCTTTCATGATGAATGTGCAAGAGCTGGGCACCGTAAAA
CGCAAGCAGTTACCGTTGAAAATCGTCTTACTCGATAACCAACGGTTAGGG
ATGGTTCGACAATGGCAGCAACTGTTTTTCCAGGAACGATATAGCGAAACC
ACCCTTACCGATAACCCCGATTTCCTCATGTTAGCCAGCGCCTTCGGCATCC
CTGGCCAACACATCACCCGTAAAGACCAGGTTGAAGCGGCACTCGACACC

ATGCTGAACAGTGATGGGCCATACCTGCTTCATGTCTCAATCGACGAACTT
GAGAACGTCTGGCCGCTGGTGCCGCCTGGTGCCAGTAATTCAGAAATGTT
GGAGAAATTATCATGATGCAACATCAGGTCAATGTATCGGCTCGCTTCAATC
CAGAAACCTTAGAACGTGTTTTACGCGTGGTGCGTCATCGTGGTTTCCACG
TCTGCTCAATGAATATGGCCGCCGCCAGCGATGCACAAAATATAAATATCGA
ATTGACCGTTGCCAGCCCACGGTCGGTCGACTTACTGTTTAGTCAGTTAAAT
AAACTGGTGGACGTCGCACACGTTGCCATCTGCCAGAGCACAACCACATC
ACAACAAATCCGCGCCTGAGCG
CAAAAGGAATATACCAGAAAAGAGACGCTTTTAGAGCGTCTTTTTTCGTTT
T
GGAGCTACTC ACACTGCTTG CCGGAATGCT GCGCGAGAAG
TTGGGTTGGG
ATATCGAACC ACAGAATATT GCACTAACAA ACGGCAGCCA GAGCGCGTTT
TTCTACTTAT
TTAACCTGTT TGCCGGACGC CGTGCCGATG GTCGGGTCAA
AAAAGTGCTG TTCCCGCTTG
CACCGGAATA CATTGGCTAT GCTGACGCCG GACTGGAAGA AGATCTGTTT
GTCTCTGCGC
GTCCGAATAT TGAACTGCTG CCGGAAGGCC AGTTTAAATA CCACGTCGAT
TTTGAGCATC
TGCATATTGG C

### Embodiment 5 Fermentation experiment of VB5 engineering bacteria

The test strains *E.coli* MG1655 *avtA:panDBs-ilvG⁺M*/*pACYC184-panBCE, E.coli MG1655 avtA:panDBl-ilvG⁺M*/*pACYC184-panBCE* and *E. coli MG1655 avtA:panDCg-ilvG⁺M*/ *pACYC184-panBCE,* were streak-inoculated on solid LB medium plates containing 34 mg/L chloramphenicol and incubated for 12 hours at 37°C. The bacterial moss on the plate was picked and inoculated into LB medium slant, and incubated at 37°C for 10-12 h. The bacterial moss on the plate was picked and inoculated into liquid LB medium, and cultured at 37°C and 220 rpm with shaking for 12 h to obtain the seed liquid. The seed liquid was inoculated into the fermentation medium in 3% inoculum, and cultured at 37°C, 220rpm with shaking.

Fermentation medium: MOPS 80 g/L, glucose 20.0 g/L, ammonium sulfate 10.0 g/L, potassium dihydrogen phosphate 2.0 g/L, magnesium sulfate heptahydrate 2.0 g/L, yeast 5.0 g/L, trace element mixed solution 5 mL/L, and the remaining amount was water. Trace element mixed solution: FeSO₄-7H₂O10 g/L, CaCl₂1.35 g/L, ZnSO₄-7H₂O2.25 g/L, MnSO₄-4H₂O0.5 g/L, CuSO₄-5H₂O1 g/L, (NH₄)₆Mo₇O₂₄-4H₂O0.106 g/L, Na₂B₄O₇-10H₂O0.23 g/L, CoCl₂-6H₂O0.48 g/L, 35% HCl10mL/L, and the remaining amount was water.

During the incubation, the pH value of the reaction system was adjusted with ammonia to maintain it at 6.8-7.0. During the incubation, samples were taken every 4 h, and the glucose content was detected by using a biosensor analyzer SBA-40D, and when the glucose content of the system was lower than 5 g/L, the glucose was supplemented to make the glucose concentration of the system reach 20 g/L. The samples were taken after 24 h incubation, and the supernatant was centrifuged at 12000g for 2 minutes, the supernatant was taken to detect the VB5 content (Table 2).

**Table2**

| engineering bacteria | Vitamins B5 ( g/L ) |
|---|---|
| *E.coli* MG1655 *avtA:panDBs-ilvG*⁺*M*/pACYC184-*panBCE* | 2.12±0.13 |
| *E.coli* MG1655 *avtA:panDB*L*-ilvG*⁺*M*/pACYC184-*panBCE* | 3.36±0.25 |
| *E.coli* MG1655 *avtA:panDCg-ilvG*⁺*M*/pACYC184-*panBCE* | 1.77±0.15 |

The present application verifies that the screened *panD* derived from *Bacillus licheniformis* has the highest activity and can significantly improve the fermentation yield of VB5 through both β-alanine whole-cell catalysis and VB5 fermentation.

The foregoing is only a preferred embodiment of the present application, and it should be noted that for a person of ordinary skill in the art, a number of improvements and embellishments may be made without departing from the principles of the present application, and these improvements and embellishments shall also be regarded as the scope of protection of the present application.

## Claims

1. An application of enhanced expression of a L-aspartate α-decarboxylase gene *panD* in the production of vitamin B5;
Wherein,the L-aspartate α-decarboxylase is derived from *Bacillus licheniformis.*

2. The application according to claim 1, wherein the L-aspartate α-decarboxylase *gene panD* has:
(I) a nucleotide sequence shown as SEQ ID No. 3; or
(II) a nucleotide sequence obtained by substituting, deleting or adding one or more bases to the nucleotide sequence shown as (I) and having the same or similar functions as the nucleotide sequence shown as (I); or
(III) a nucleotide sequence having at least 80% homology to the nucleotide sequence shown as (I) or (II).

3. The application according to claim 1 or 2, further comprising:
(1) inserting a strong promoter and/or a strong RBS into the *avtA* gene, wherein the strong promoter is PPL and the strong RBS is BCD2; and/or
(2) expressing an *ilvGM* gene derived from E. coli BL21; and/or
(3) a *panB* gene, a *panC* gene and/or a *panE* gene with increased copy number.

4. An expression vector, comprising a L-aspartate α-decarboxylase gene *panD*;
the L-aspartate α-decarboxylase is derived from *Bacillus licheniformis.*

5. The expression vector according to claim 4, further comprising:
(I) a strong promoter and/or a strong RBS; and/or
(II) an *ilvGM* gene derived from E. coli BL21; and/or
(III) a *panB* gene, a *panC* gene and/or a *panE* gene with increased copy number.

6. A host, wherein a L-aspartate α-decarboxylase gene *panD* derived from *Bacillus licheniformis* is expressed.

7. The host, according to claim 6, further comprising:
(I) a strong promoter and/or a strong RBS; and/or
(II) an *ilvGM* gene derived from E. coli BL21; and/or
(III) a *panB* gene, a panC gene and/or a *panE* gene with increased copy number.

8. The host according to claim 6 or 7, wherein the expression vector according to claim 4 or 5 is transfected or transformed;
preferably, the host is derived from E. coli,
more preferably, the host is derived from E. coli K12, and
more preferably, the host is derived from E. coli K12 MG1655 strain.

9. The application of the expression vector according to claim 4 or 5, the host according to any one of claims 6 to 8, in the production of vitamin B5.

10. A method for the production of vitamin B5, wherein the host according to any one of claims 6 to 8 is used as a fermentation strain without the addition of β-alanine, fermented, the fermentation broth is collected, the supernatant is centrifuged and vitamin B5 is obtained.
